# EUROPEAN PATENT APPLICATION

(11) **EP 1 431 386 A1**
(43) Date of publication of application: **23.06.2004**
(21) Application number: 02775248.4
(22) Date of filing: 27.09.2002
(51) Int. Cl.: C12N 1/00

(54) **METHOD OF AMPLIFYING DNA CHIP SIGNALS**

(30) Priority: 28.09.2001 JP 2001303816
(71) Applicant: SANKO JUNYAKU CO., LTD., Tokyo 101-0032 (JP)
(72) Inventor: USUI, Mitsugu, Abiko-shi, Chiba 270-1176 (JP); MITSUKA, Mari, Kashiwa-shi, Chiba 277-0022 (JP); HAKII, Chikako, Kashiwa-shi, Chiba 277-0841 (JP)
(74) Representative: Grünecker, Kinkeldey, Stockmair & Schwanhäusser Anwaltssozietät
(86) International application number: PCT/JP2002/010034
(87) International publication number: WO 2003/029441

(57) **Abstract**

There is provided a signal amplification method for a DNA chip which can establish efficient signal amplification for a target gene captured on the DNA chip by the PALSAR method and can establish a simple detection by contriving design of a pair of HCPs to be used in the PALSAR method. Sensitivity for detection of the target gene on the DNA chip is improved by making use of a self-assembly reaction which forms a double-stranded self-assembly substance having a regular higher-order structure of oligonucleotides.

## Description

### Technical Field:

The present invention relates to a signal amplification method for a DNA chip, and more particularly, relates to a signal amplification method for a gene which can improve sensitivity for detection of a target gene on a DNA chip by making use of a pair of oligonucleotides forming a self-assembly substance, wherein the DNA chip comprises a support or a substrate to bind a gene for capturing the target gene (hereinafter referred to as a support), and the support is of micro plate type, slide glass type, microparticle type, electroconductive substrate type or the like (hereinafter may be generically referred to as a DNA chip).

### Background Art:

DNA chips are used for detection of genes in which a number of different DNA probes are densely immobilized on a support of micro plate type, slide glass type, microparticle type, or electroconductive solid substrate type, wherein the support surface is specially processed, followed by hybridization of nucleic acids (targets) (Refer to Judy, M., Geoffrey, W. Hybridization of Nucleic Acids Immobilized on Solid Supports. *Analytical Biochemistry,* 138,267-284,1984, Marshall, A., Hodgson, J. DNA chips: an array of possibilities. *Nat Biotechnol.* 16,27-31,1998, JP4135498, JP4135500, US5952172A, US6346387B1 and US6180346B1), and signals from nucleic acids (targets) are measured.

Currently, this technique has been utilized for analysis of gene expression and detection of variant genes or the like. However, there have been problems that, in comparison with conventionally used Southern blotting (Southern, E. M. Detection of specific sequences among DNA fragments separated by gel electrophoresis. *J. Mol. Biol.,* 98,503-517,1975), sensitivity for detection of the DNA chip method is one tenth as low as that of the Southern blotting (Masaaki Muramatsu et al. DNA Microarray and the latest PCR Method. 85-86, 2000, Shujunsha Co. Ltd.) and that time required for reaction in the former is longer than that in the latter.

In addition, there are methods in which a target gene is amplified beforehand to enhance the sensitivity for detection of a target gene using such methods for amplifying gene as the LCR method for amplifying gene with an enzyme named thermostable DNA ligase (USP 5,792,607), the PCR method for amplifying gene with an enzyme named thermostable DNA polymerase (Saiki, R., S. Scharf, F. Faloona, et al. Enzymatic amplification of β-globin genomic sequence and restriction site analysis for diagnosis of sickle cell anemia. *Science.* 230,1350-1354(1985)) and the like. These methods require, however, complicated operations and a high cost.

In view of the above problems, the present inventors have proposed a novel, isothermal nucleic acid amplification method without using any enzyme (USP 6,261,846, JP 3267576 and EP 1,002,877A). This method utilizes a pair of oligonucleotides each comprising three regions (HoneyComb Probe; referred to as an HCP hereinafter) in which the three respective regions of a first HCP and a second HCP are designed to be composed of base sequences complementary to each other so that only one region of the first HCP may be hybridized with one region of the second HCP when the two oligonucleotides are reacted. This design makes it possible for a plurality of pairs of HCPs to hybridize to each other and form an assembly substance by a self-assembly reaction of HCPs when they are reacted to each other (Probe alternation link self-assembly reaction; this method for the formation of an assembly substance by the self-assembly reaction of HCPs is referred to as a PALSAR method hereinafter).

### Disclosure of the Invention:

In view of the present state of the prior art described above, as a result of the present inventors' diligent study for improving the sensitivity for detection of a DNA chip using the above PALSAR method, the present inventors have attained the present invention.

The object of the present invention is to provide a signal amplification method for a DNA chip which can establish efficient signal amplification for a target gene captured on the DNA chip by the PALSAR method and can establish simple detection by contriving design of a pair of HCPs to be used in the PALSAR method.

In order to solve the above problems, a signal amplification method for a DNA chip according to the present invention improves sensitivity for detection of a target gene on the DNA chip by making use of a self-assembly reaction which forms a double-stranded self-assembly substance having a regular higher-order structure of oligonucleotides.

For the self-assembly reaction described above, firstly, it is possible to utilize the self-assembly reaction which comprises the steps of: providing a plurality of pairs of oligonucleotide-probes comprising n (n≧3) regions, each region of a first probe of the pair of probes being complementary to each region of a second probe of the pair of probes; and hybridizing the pairs of oligonucleotide-probes such that the first probes and the second probes cross each other in alternation, wherein the oligonucleotide-probes are self-assembled to form the double-stranded self-assembly substance.

For the self-assembly reaction described above, secondly, it is possible to utilize the self-assembly reaction which comprises the steps of: providing a first group and a second group, the first group including a plurality of pairs of dimer-forming probes containing a pair of an oligonucleotide No.1 and an oligonucleotide No.2, each oligonucleotide having three regions of a 3' side region, a mid-region and a 5' side region, in which the mid-regions thereof have base sequences complementary to each other to form a dimer-probe, and the 3' side regions and the 5' side regions thereof have base sequences not complementary to each other, the second group including a plurality of pairs of cross-linking probes containing a pair of an oligonucleotide No.3 and an oligonucleotide No.4, each oligonucleotide having two regions of a 3' side region and a 5' side region, in which the 3' side regions and the 5' side regions thereof have base sequences not complementary to each other, and the pairs of the cross-linking probes having base sequences capable of cross-linking the dimer probes formed from the dimer-forming probes; and hybridizing the probes (the dimer-forming probes and the cross-linking probes are collectively referred to as probes), wherein the oligonucleotides are self-assembled to form the self assembly substance.

It is possible to use the probes, wherein the base sequences of the probes are made complementary to each other in the following respective pairs: the 3' side region of the oligonucleotide No.1 in the first group and the 3' side region of the oligonucleotide No.3 in the second group; the 5' side region of the oligonucleotide No. 2 in the first group and the 5' side region of the oligonucleotide No.4 in the second group; the 3' side region of the oligonucleotide No.4 in the second group and the 3' side region of the oligonucleotide No.2 in the first group; and the 5' side region of the oligonucleotide No.3 in the second group and the 5' side region of the oligonucleotide No.1 in the first group.

In addition, it is possible to use the probes, wherein the base sequences of the probes are made complementary to each other in the following respective pairs: the 3' side region of the oligonucleotide No.1 in the first group and the 3' side region of the oligonucleotide No.3 in the second group; the 5' side region of the oligonucleotide No.2 in the first group and the 5' side region of the oligonucleotide No.3 in the second group; the 3' side region of the oligonucleotide No.2 in the first group and the 3' side region of the oligonucleotide No.4 in the second group; and the 5' side region of the oligonucleotide No.1 in the first group and the 5' side region of the oligonucleotide No.4 in the second group.

It is preferable that the above DNA chip comprises a support to bind a gene for capturing the target gene, and the support is of micro plate type, slide glass type, microparticle type or electroconductive substrate type or the like. As materials for the above micro plate type and microparticle type supports, plastics, polystyrene or the like may be used. Materials such as glass, plastics or the like may be used in the slide glass type support. Gold electrode, ITO electrode (indium oxide electrode) or the like may be used in the electroconductive substrate type support.

A single-stranded DNA and/or RNA may be used as the target gene.

A double-stranded DNA and/or RNA may be used as the target gene.

SNPs (single nucleotide polymorphisms) may be used as the target gene.

It is preferable that a base sequence of the oligonucleotides used for the self-assembly reaction is made, in advance, complementary to a base sequence of the target gene.

It is preferable that in order to link the target gene to the self-assembly substance, there is used a joint-probe which contains a first region complementary to a base sequence of the target gene and a second region complementary to a base sequence of the oligonucleotides used for formation of the self-assembly substance.

By hybridizing a labeled probe to the self-assembly substance formed by the self-assembly reaction of the oligonucleotides and bound to the target gene, the presence of the self-assembly substance can be detected.

As the labeled probe, a probe labeled with an enzyme of color generation type, an enzyme of luminescence generation type, a radioisotope or the like may be used.

It is possible to detect the presence of the self-assembly substance by: adding a fluorescent substance capable of binding to a nucleic acid to the self-assembly substance; and measuring a photochemical change of the fluorescent substance.

It is also possible to detect the presence of the self-assembly substance by: labeling in advance at least one of the oligonucleotides forming the self-assembly substance with a fluorescent substance; and measuring a photochemical change of the fluorescent substance.

In addition, it is possible to detect the presence of the self-assembly substance by: labeling in advance at least one of the oligonucleotides forming the self-assembly substance with a radioisotope; and detecting the radioisotope.

Furthermore, it is possible to detect the presence of the self-assembly substance by: labeling in advance at least one of the oligonucleotides forming the self-assembly substance with an enzyme of color generation type or an enzyme of luminescence generation type; and measuring a photochemical change due to the enzyme.

The oligonucleotides described above are comprised of at least one base selected from the group consisting of DNA, RNA, PNA and LNA.

### Brief Description of the Drawings:

Fig. 1 is a schematic diagram showing in principle Step 100 in the first to fourth embodiments in step orders of the signal amplification method of the present invention;
Fig. 2 is a schematic diagram showing in principle Step 102 in the first to fourth embodiments in step orders of the signal amplification method of the present invention;
Fig. 3 is a schematic diagram showing in principle Step 110 in the first embodiment in step orders of the signal amplification method of the present invention;
Fig. 4 is a schematic diagram showing in principle Step 114 in the first embodiment in step orders of the signal amplification method of the present invention;
Fig. 5 is a schematic diagram showing in principle Step 122 in the second embodiment in step orders of the signal amplification method of the present invention;
Fig. 6 is a schematic diagram showing in principle Step 124 in the second embodiment in step orders of the signal amplification method of the present invention;
Fig. 7 is a schematic diagram showing in principle Step 130 in the third embodiment in step orders of the signal amplification method of the present invention;
Fig. 8 is a schematic diagram showing in principle Step 132 in the third embodiment in step orders of the signal amplification method of the present invention;
Fig. 9 is a schematic diagram showing in principle Step 134 in the third embodiment, in step orders of the signal amplification method of the present invention;
Fig. 10 is a schematic diagram showing in principle Step 144 in the fourth embodiment in step orders of the signal amplification method of the present invention;
Fig. 11 is a photograph showing the results of Examples 1 to 3 and Comparative Examples 1 to 3;
Fig. 12 is a photograph showing the results of Examples 4 to 6 and Comparative Examples 4 to 6;
Fig. 13 is a photograph showing the results of Examples 7 to 9 and Comparative Examples 7 to 9;
Fig. 14 is a photograph showing the results of Examples 10 to 12 and Comparative Examples 10 to 12;
Fig. 15 is a photograph showing the results of Examples 13 to 15 and Comparative Examples 13 to 15;
Fig. 16 is a photograph showing the results of Examples 16 to 18 and Comparative Examples 16 to 18; and
Fig. 17 is a photograph showing the results of Experimental Examples 1 to 4.

### Best Mode for Carrying Out the Invention:

Several embodiments of the present invention will be described hereinafter with reference to the accompanying drawings. It goes without saying, however, that these embodiments are merely illustrative, and various modifications may be made without departing from the technical idea of the present invention.

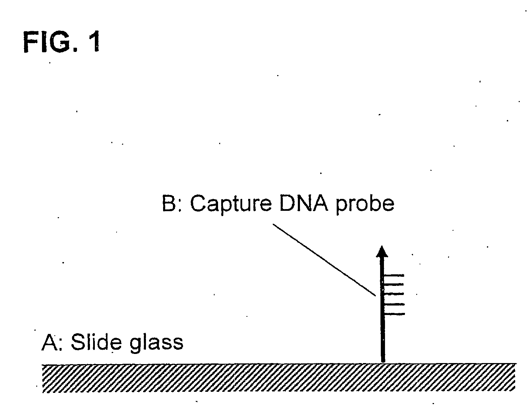

Figs. 1 to 4 are schematic diagrams showing in principle a first embodiment in step orders of the signal amplification method of the present invention. The first embodiment is a signal amplification method for a DNA chip by making use of the PALSAR method using a pair of oligonucleotide-probes (that is, a pair of HCPs; HCP-1 and HCP-2) ach composed of three regions complementary to each other, capable of self-assembling themselves to form a assembly substance, wherein the pair of HCPs contain a region complementary to a target gene, and are previously labeled with a fluorescent substance.

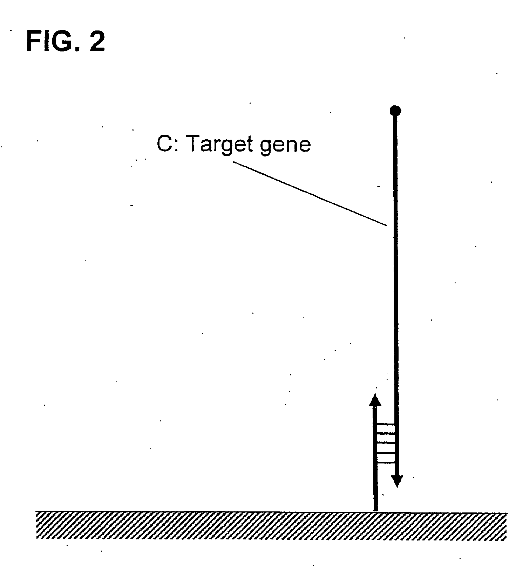

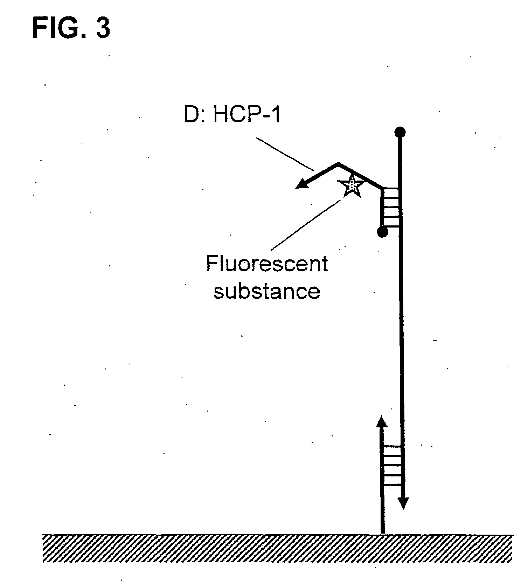

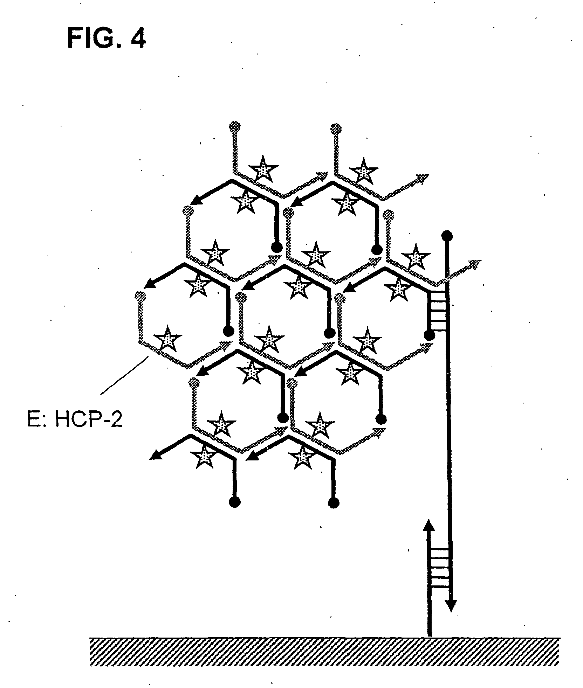

As shown in Fig. 1, a capture DNA probe (Symbol B) having a region complementary to the target gene is bound onto a slide glass (Symbol A) used as a support (Step 100). Next, the target gene (Symbol C) is captured as shown in Fig. 2 (Step 102), and then as shown in Fig. 3, one of the pair, HCP-1 (Symbol D), having a region complementary to the target gene, is bound thereon (Step 110), wherein the pair of probes can self-assemble themselves to form a assembly substance and are labeled with a fluorescent substance. As shown in Fig. 4, the other oligonucleotide-probe of the pair, HCP-2 (Symbol E), was added to this DNA chip shown in Fig.3 (Step 112), and a self-assembly substance is formed by self-assembly reaction, thereby permitting the signal to be amplified (Step 114). It should be noted that Step 110 and Step 112 can be carried out simultaneously as well.

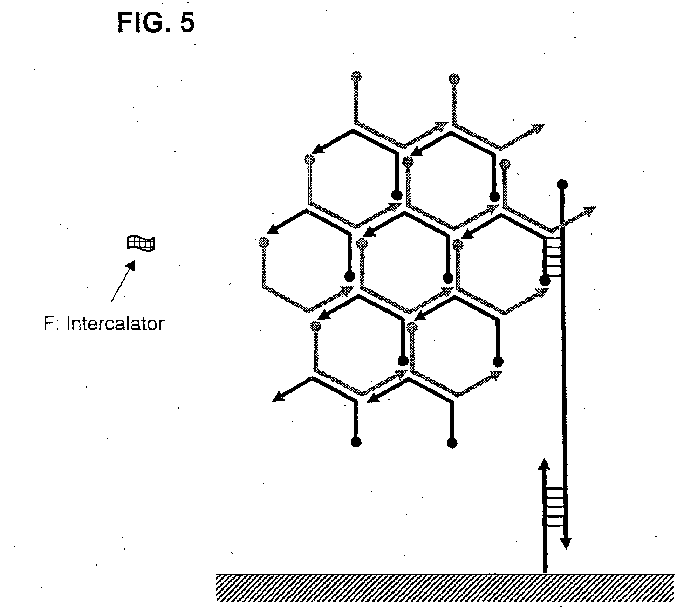

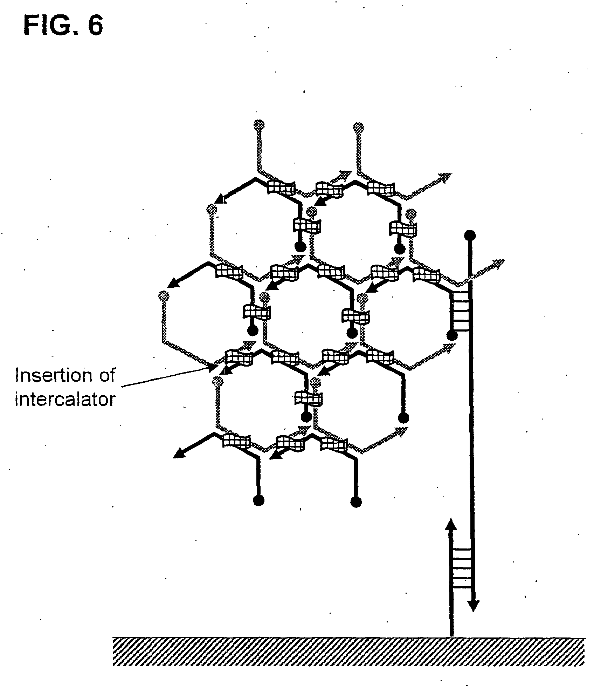

Fig. 5 and Fig. 6 are schematic diagrams showing in principle a second embodiment in step orders of the signal amplification method of the present invention. The second embodiment is a signal amplification method for a DNA chip by making use of the PALSAR method using a pair of HCPs, wherein the pair of HCPs contain a region complementary to a target gene and are not labeled with a fluorescent substance.

In a similar manner to the first embodiment described above, Step 100 and Step 102 are carried out. Then, the pair of HCPs are added to form a self-assembly substance (Step 120). As shown in Fig. 5, an intercalator (Symbol F) is inserted into the formed self-assembly substance (Step 122), thereby permitting the signal to be amplified (Step 124), as shown in Fig. 6. It should be noted that Step 120 and Step 122 can be carried out simultaneously as well.

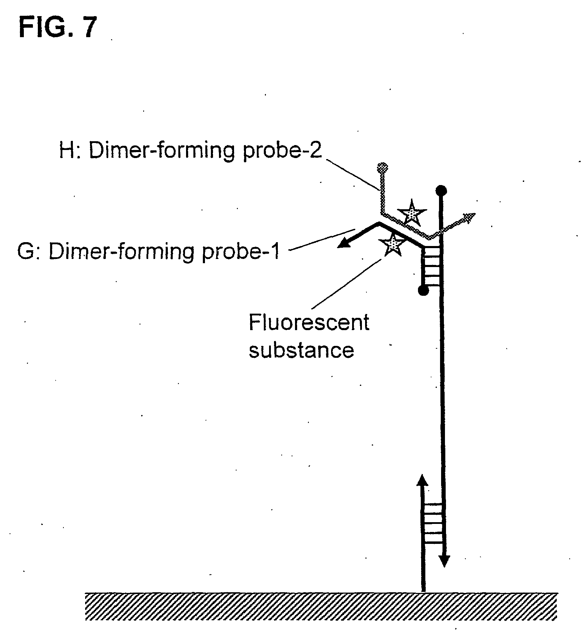

Figs. 7 to 9 are schematic diagrams showing in principle a third embodiment in step orders of the signal amplification method of the present invention. The third embodiment is a signal amplification method for a DNA chip by making use of the PALSAR method using a pair of dimer-forming probes capable of forming a dimer themselves and a pair of cross-linking probes capable of cross-linking the dimer formed from the dimer-forming probes, wherein the pair of dimer-forming probes (dimer-forming probe-1 and probe-2) contain a region complementary to a target gene and are previously labeled with a fluorescent substance.

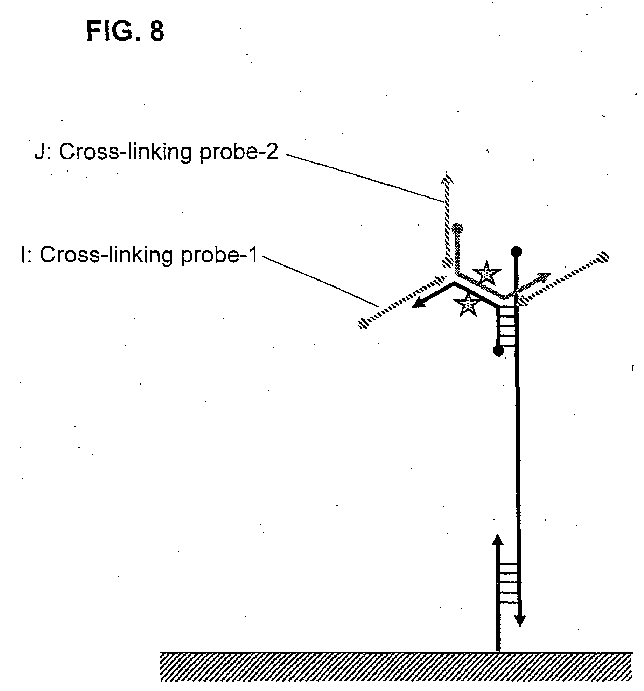

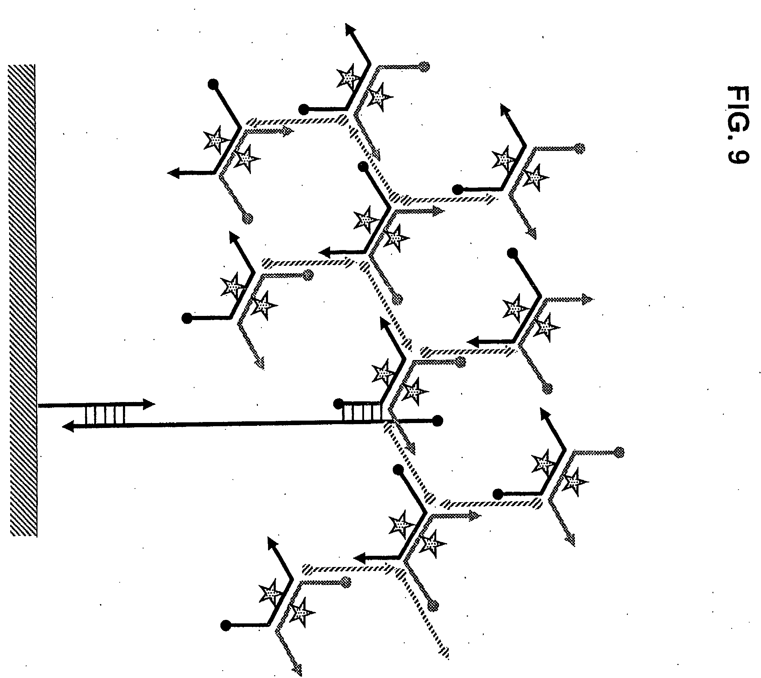

In a similar manner to the first embodiment described above, Step 100 and Step 102 are carried out. Then, the pair of the dimer-forming probe-1 and probe-2 (Symbols G and H) containing a region complementary to the target gene and capable of forming a dimer themselves are hybridized to the target gene (Step 130), as shown in Fig. 7. Then, the cross-linking probe-1 and probe-2 (Symbols I and J) having regions complementary to the dimer-forming probe-1 and probe-2 which have formed dimer are hybridized thereon (Step 132), as shown in Fig 8, and a self-assembly substance is formed by self-assembly reaction of the dimer-forming probes and the cross-linking probes, thereby permitting the signal to be amplified (Step 134), as shown in Fig 9. It should be noted that Step 130 and Step 132 can be carried out simultaneously as well.

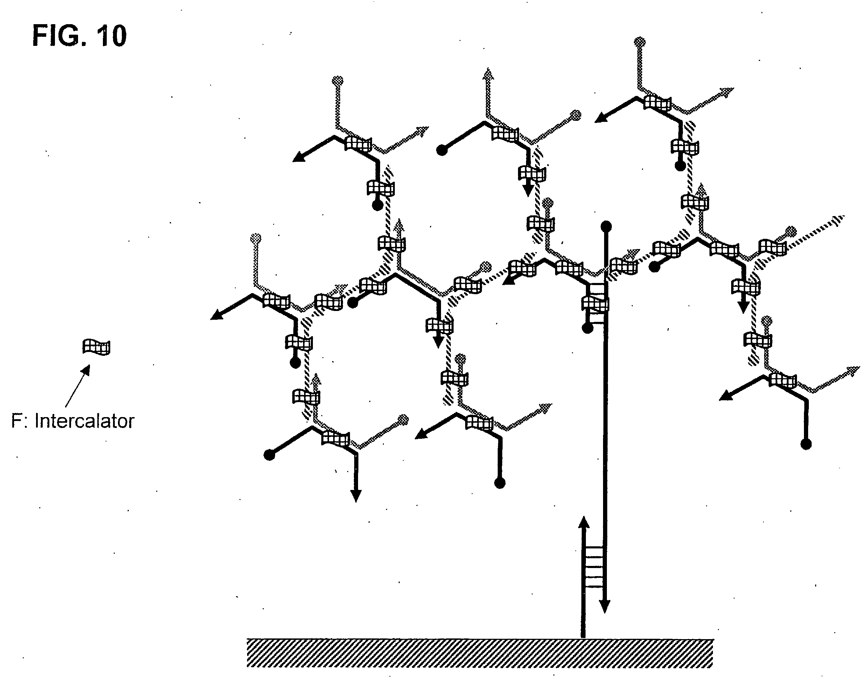

Fig. 10 is a schematic diagram showing in principle a fourth embodiment in step orders of the signal amplification method of the present invention. The fourth embodiment is a signal amplification method for a DNA chip by making use of the PALSAR method using a pair of dimer-forming probes capable of forming a dimer themselves and a pair of cross-linking probes capable of cross-linking the dimer formed from the dimer-forming probes, wherein the pair of dimer-forming probes (dimer-forming probe-1 and probe-2) contain a region complementary to a target gene. In this embodiment, neither the dimer-forming probes nor the cross-linking probes are labeled.

In a similar manner to the first embodiment described above, Step 100 and Step 102 are carried out. Then, the pair of the dimer-forming probes and the pair of the cross-linking probes are added to form a self-assembly substance (Step 140). As shown in Fig. 10, an intercalator (Symbol F) is inserted into the formed self-assembly substance (Step 142), thereby permitting the signal to be amplified (Step 144). It should be noted that Step 140 and Step 142 can be carried out simultaneously as well.

In the signal amplification method of the present invention, as labeled materials for detection, for example, radioisotopes such as ¹²⁵I, ³²P and the like, luminescent substances such as digoxigenin, acridinium ester or the like, fluorescent substances such as Cy3, Cy5 or the like, biotin for utilizing fluorescent substances such as 4-methylumbelliferyl phosphate or the like, and a donor fluorescent dye and an acceptor fluorescent dye for utilizing fluorescent resonance energy transfer (FRET) may be previously added to a pair of oligonucleotide probes to detect a target gene.

In addition, a dye having a property of binding to nucleic acids may be added for the purpose of detecting the target gene. It is preferable to detect the target gene by using a fluorescent substance having a property of binding to nucleic acids such as an intercalator, as shown in Fig. 5, Fig. 6 and Fig. 10. Although there is no limitation for the fluorescent substance as long as it is a fluorescent substance having the property of binding to nucleic acids, for example, SYBR Green I stain, SYBR Green II stain, SYBR Green Gold stain, Vistra Green stain, Gelstar stain, Radlant Red stain, PicoGreen, RiboGreen, OllGreen, Hoechst 33258 (Bis-Benzimide), Propidium Iodide, YO-PRO-1 Iodide, YO-PRO-3 Iodide (All the foregoing are made by Molecular Probes, Inc.), ethidium bromide, Distamycin A, TOTO, Psoralen, Acridine Orange, AOAO (homodimer) and the like can be used.

Although the nucleic acids constituting a pair of the oligonucleotide-probes described above are generally composed of DNA or RNA, analogs of nucleic acids are also acceptable. These include, for example, Peptide Nucleic Acid (PNA, WO 92/20702), and Locked Nucleic Acid (LNA, Koshkin AA et al. *Tetrahedron* 1998. 54, 3607-3630., Koshkin AA et al. *J. Am. Chem. Soc.* 1998. 120, 13252-13253., Wahlestedt C et al. *PNAS.* 2000. 97, 5633-5638.). A pair of the oligonucleotide-probes are composed of the same kind of nucleic acids in general, while a pair of DNA probe and RNA probe may also be usable. In other words, the kinds of nucleic acids for use in the probes are selected from DNA, RNA and analogs of nucleic acids (for example, PNA, LNA and the like). Further, nucleic acids in one probe may not be necessarily composed of one kind of nucleic acid, for example, only DNA. An oligonucleotide-probe (a chimera probe) composed of such as DNA and RNA may be used, if necessary, and these are also included in the scope of the present invention.

The length of each complementary base sequence region in the oligonucleotide-probes is at least 5 bases, preferably 10 to 100 bases, and more preferably 15 to 30 bases in terms of the number of bases.

These probes may be synthesized by known methods. For example, DNA probes may be synthesized by phosphoamidite method using the DNA synthesizer Model 394 of Applied Biosystems Inc. These are also synthesized by any one of other available methods such as phosphotriester method, H-phosphonate method, thiophosphonate method and the like.

In the present invention, an assembly substance is formed by using a pair of HCPs, or a pair of dimer-forming probes and a pair of cross-linking probes, each having a region complementary to a target gene captured by a DNA chip. The number of the oligonucleotide-probes to be used in the above purpose is not particularly limited, but is generally in the range of 10² to 10¹⁵. The composition and the concentration of a buffer solution used for the reaction are not particularly limited, and conventional buffer solutions used for amplification of nucleic acids may preferably be used. The pH may be also suitable in the common range, and it is preferable to use in the range of pH 7.0 to pH 9.0. The reaction temperature ranges from 40 to 80 °C, and preferably ranges from 55 to 65 °C.

As a sample for measuring a target gene (DNA and/or RNA) in the present invention, any sample that would potentially contain the corresponding nucleic acids may be used. The target genes may be either prepared or isolated appropriately from the sample, and is not particularly limited. For example, there are given as the samples ones derived from an organism such as blood, serum, urine, feces, cerebrospinal fluid, tissue fluid, cell cultures or the like, ones which may contain or be infected with viruses, bacteria, fungi or the like, and so on. Nucleic acids such as DNA and/or RNA in which a target gene in a sample has been amplified by a known method can also be used.

### EXAMPLES

The present invention will hereinafter be described in more specific manner by way of the following examples which should be construed as illustrative rather than restrictive.

### 1. Materials

The following are oligonucleotide-probes used in the examples:
[1] Probe 1; Capture DNA probe-1a 5'-NH₂-AAAAAAGCGG GAAATCGTGC GTGACATTAA-3'
[2] Probe 2; Target gene-1 
5'-CGTGGCCATC TCTTGCTCGA AGTCCAGGGC GACGTAGCAC AGCTT
CTCCT TAATGTCACG CACGATTTCC CGCTCGGCCG-3'
[3] Probe 3; HCP-1 
5'-CCTGGACTTC GAGCAAGAGA TGGCCGGCAC CACCATGTAC CCTGG
CATTG GTCCACCGCA AATGCTTCTA GGCGG-3'
[4] Probe 4; HCP-2 
5'-GGCCATCTCT TGCTCGAAGT CCAGGCAATG CCAGGGTACA TGGTG
GTGCC CCGCCTAGAA GCATTTGCGG TGGAC-3'
[5] Probe 5; HCP-3 
5'-CCCTGGACTT CGAGCAAGAG CGTGGACATC CGCAAAGACC GCAGG
AGTAT GACGAGTCCG-3'
[6] Probe 6; HGP-4 
5'-CTCTTGCTCG AAGTCCAGGG GGTCTTTGCG GATGTCCACG CGGAC
TCGTC ATACTCCTGC-3'
[7] Probe 7; Dimer-forming probe-1 
5'-GACTTCGAGC AAGAGATGGC CCTGATAGGG TGCTTGCGAG CCATT
GGCAA TGAGCGGTTC-3'
[8] Probe 8; Dimer-forming probe-2 
5'-GAGCATCCCC CAAAGTTCAC CTCGCAAGCA CCCTATCAGG GTGGC
ATCCA CGAAACTACC-3'
[9] Probe 9; Cross-linking probe-1 5'-GCCATCTCTT GCTCGAAGTC GAACCGCTCA TTGCCAATGG-3'
[10] Probe 10; Cross-linking probe-2 5'-GTGAACTTTG GGGGATGCTC GGTAGTTTCG TGGATGCCAC-3'
[11] Probe 11; Capture DNA probe-2a 
5'-NH2-GATCAGACAC TTCAAGGTCT CTGGCGCTTT AAGATTAGCC TG
CGTCCGAT-3'
[12] Probe 12; Capture DNA probe-1b 5'-NH2-AAAAAAGCGG GAAATCGTGC GTGACATTAA GGAGAAGCTG-3'
[13] Probe 13; Target gene-2 
5’-TGCTTCTTCT GTGGGTAACG TCCAGTTAAT CAGCTCTTAA CCTATC
AACC CTCCAACTAG CTAATCGGAC GCAGGCTAAT CTTAAAGCGC CAG
GCCCGAA-3’
[14] Probe 14; HCP-5 
5'-GAGGGTTGAT AGGTTAAGAG CTGGTAGAGA TCGGAAGGAA CACCA
GCAGC AAACGCGATA AGTTGACCG-3'
[15] Probe 15; HCP-6 
5'-CAGCTCTTAA CCTATCAACC CTCCTGGTGT TCCTTCCGAT CTCTAC
CGGT CAACTTATCG CGTTTGCTG-3'
[16] Probe 16; Capture DNA probe-2b

5'-SH-GATCAGACAC TTCAAGGTCT CTGGCGCTTT AAGATTAGCC TGC
GTCCGAT-3'

As a hybrid solution, the following solution was used: 0.8 µg/µL poly-dA, 0.1 µg/µL yeast t-RNA, 0.15 µg/µL human Cot-I DNA, 5×Denhardt's solution, 0.1 mg/mL Salmon sperm DNA, 0.2% SDS, 6×SSC.

### (Examples 1 to 3 and Comparative Examples 1 to 3)

### 1.Object

The effect of signal amplification was examined by using a pair of HCPs (HCP-1 and HCP-2) labeled with Cy3.

### 2. Materials

### (Examples 1 to 3)

a) As a capture probe, the capture DNA probe-1a having a region complementary to the target gene-1 and prepared at 500 ng/µL (Example 1), 50 ng/µL (Example 2), or 5 ng/µL (Example 3) was used, respectively.
b) As a primary hybridization solution, 50 ng of the target gene-1 was dissolved in 10 µL of the hybrid solution, followed by heating for 2 minutes at 95°C to prepare a primary hybridization solution A.
c) As a secondary hybridization solution, 25 pmoles of Cy3-labeled HCP-1 and 25 pmoles of Cy3-labeled HCP-2 were dissolved in 10 µL of the hybrid solution, followed by heating for 2 minutes at 95°C to prepare a secondary hybridization solution A.

### (Comparative Examples 1 to 3)

a) As a capture probe, the capture DNA probe-1a prepared at 500 ng/µL (Comparative Example 1), 50 ng/µL (Comparative Example 2), or 5 ng/µL. (Comparative Example 3) was used, respectively.
b) As a primary hybridization solution, the above primary hybridization solution A was used.
c) As a secondary hybridization solution, 25 pmoles of Cy3-labeled HCP-1 was dissolved in 10 µL of the hybrid solution, followed by heating for 2 minutes at 95°C to prepare a secondary hybridization solution B.

### 3. Methods

The capture probe was applied onto a slide glass by spotting to make the probe immobilized. Then, 25 µL of the primary hybridization solution was dropped onto the spot on the slide glass. It was covered with a cover glass, and left standing for 24 hours at 42°C in a hybridization chamber. After that, the slide glass was taken out, and the cover glass was removed immersing in a solution of 2×SSC and 0.1% SDS at room temperature, followed by rinsing of the slide glass with the solution of 2×SSC and 0.1% SDS. In addition, the slide glass was left standing in solutions of 1×SSC and 0.2×SSC for 2 minutes, respectively, at room temperature and dried by spraying air.

Then 10 µL of the secondary hybridization solution was dropped to the spot on the slide glass. It was covered with a cover glass, and left standing for 16 hours at 42°C in a hybridization chamber. After that, the slide glass was taken out, and the cover glass was removed immersing in the solution of 2×SSC and 0.1% SDS at room temperature, followed by rinsing of the slide glass with the solution of 2×SSC and 0.1% SDS. Further, the slide glass was left standing in solutions of 1×SSC and 0.2×SSC for 2 minutes, respectively, at room temperature and dried by spraying air.

### 4. Detection Method

The above prepared slide glass was observed with a fluorescent microscope (580 nm).

### 5. Results

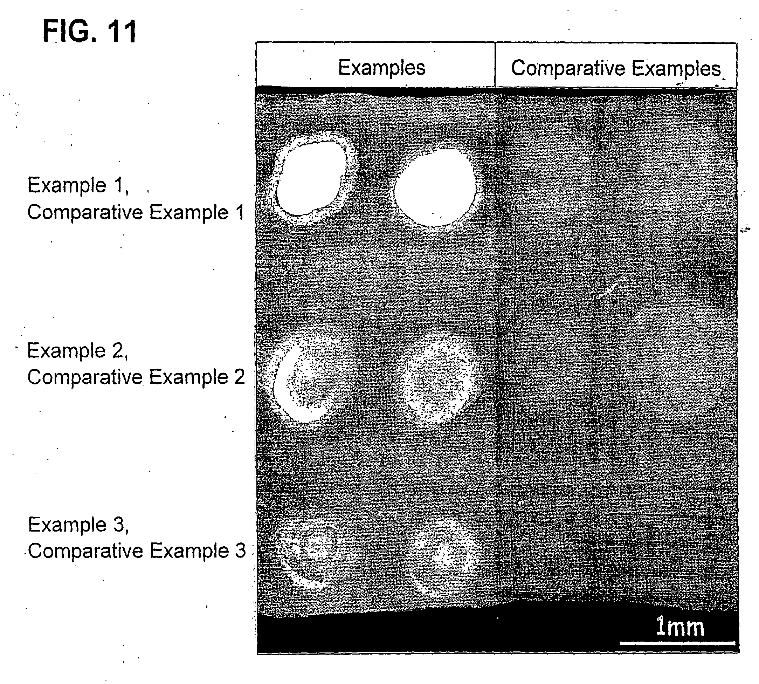

The results of Examples 1 to 3 and Comparative Examples 1 to 3 are shown in Fig. 11. The fluorescence intensities of Examples 1 to 3 in which self-assembly substances were formed strikingly increased in comparison with those of Comparative Examples 1 to 3 in which self-assembly substances were not formed, as shown in Fig. 11. By making use of the reaction forming self-assembly substance with the use of the pair of the Cy3-labeled HCPs, a signal of the DNA chip was remarkably amplified depending on the concentrations of the capture probe.

### (Examples 4 to 6 and Comparative Examples 4 to 6)

### 1. Object

The effect of signal amplification with a pair of HCPs (HCP-1 and HCP-2) was detected by using SYBR Green I.

### 2. Materials

### (Examples 4 to 6)

a) As a capture probe, the capture DNA probe-1a prepared at 500 ng/µL (Example 4), 50 ng/µL (Example 5), or 5 ng/µL. (Example 6) was used, respectively.
b) As a primary hybridization solution, the above primary hybridization solution A was used.
c) As a secondary hybridization solution, 25 pmoles of HCP-1 and 25 pmoles of HCP-2 were dissolved in 10 µL of the hybrid solution, followed by heating for 2 minutes at 95°C to prepare a secondary hybridization solution C.

### (Comparative Examples 4 to 6)

a) As a capture probe, the capture DNA probe-1a prepared at 500 ng/µL (Comparative Example 4), 50 ng/µL (Comparative Example 5), or 5 ng/µL (Comparative Example 6) was used, respectively.
b) As a primary hybridization solution, the above primary hybridization solution A was used.
c) As a secondary hybridization solution, 25 pmoles of HCP-1 was dissolved in 10 µL of the hybrid solution, followed by heating for 2 minutes at 95°C to prepare a secondary hybridization solution D.

### 3. Methods

Each Experiment was carried out on the procedure and conditions both similar to those in Examples 1 to 3 and Comparative Examples 1 to 3.

### 4. Detection Method

An undiluted solution of SYBR Green I was diluted with 0.5xTBE at a dilution ratio of 1000 times, and the diluted solution was dropped onto the spot on the slide glass and left standing for 3 minutes at room temperature. The slide glass was rinsed three times with 0.5×TBE at room temperature, dried by spraying air and observed with a fluorescent microscope (475 nm).

### 5. Results

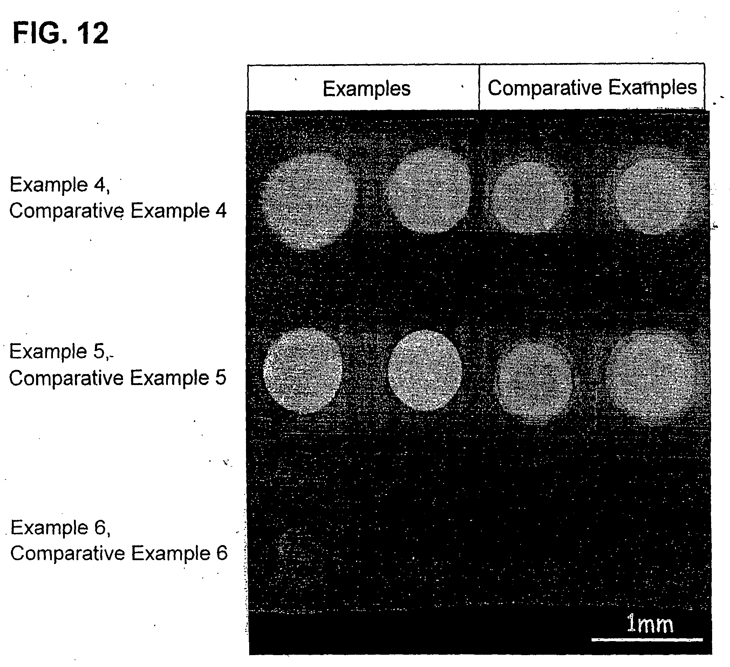

The results of Examples 4 to 6 and Comparative Examples 4 to 6 are shown in Fig. 12. The fluorescence intensities of Examples 4 to 6 in which self-assembly substances were formed strikingly increased in comparison with those of Comparative Examples 4 to 6 in which self-assembly substances were not formed, as shown in Fig. 12. By making use of reaction forming self-assembly substance with the pair of the HCPs and by detecting with SYBR Green I, a signal of the DNA chip was remarkably amplified depending on the concentrations of the capture probe.

### (Examples 7 to 9 and Comparative Examples 7 to 9)

### 1. Object

The effect of signal amplification with a pair of HCPs (HCP-1 and HCP-2) was detected by using PicoGreen.

### 2. Materials

### (Examples 7 to 9)

a) As a capture probe, the capture DNA probe-1a prepared at 500 ng/µL (Example 7), 50 ng/µL (Example 8), or 5 ng/µL (Example 9) was used, respectively.
b) As a primary hybridization solution, the above primary hybridization solution A was used.
c) As a secondary hybridization solution, the above secondary hybridization solution C was used.

### (Comparative Examples 7 to 9)

a) As a capture probe, the capture DNA probe-1a prepared at 500 ng/µL (Comparative Example 7), 50 ng/µL (Comparative Example 8), or 5 ng/µL (Comparative Example 9) was used, respectively.
b) As a primary hybridization solution, the above primary hybridization solution A was used.
c) As a secondary hybridization solution, the above secondary hybridization solution D was used.

### 3. Methods

Each experiment was carried out on the procedure and conditions both similar to those described in item 3 of Examples 1 to 3 and Comparative Examples 1 to 3.

### 4. Detection Method

A undiluted solution of PicoGreen was diluted with TE at a dilution ratio of 200 times, and the diluted solution was dropped onto the spot on the slide glass and left standing for 30 minutes at room temperature. The slide glass was rinsed three times with 0.5×TBE at room temperature, dried by spraying air and observed with a fluorescent microscope (475 nm).

### 5. Results

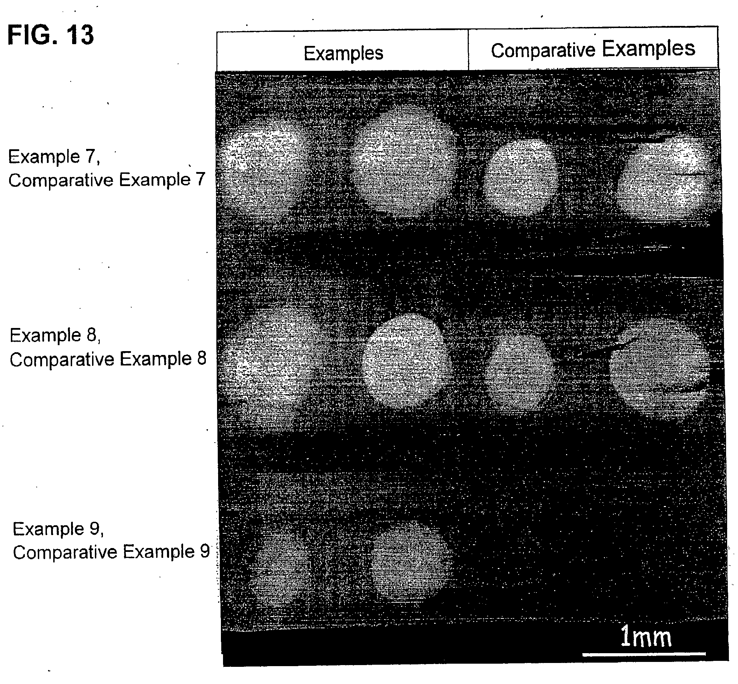

The results of Examples 7 to 9 and Comparative Examples 7 to 9 are shown in Fig. 13. The fluorescence intensities of Examples 7 to 9 in which self-assembly substances were formed strikingly increased in comparison with those of Comparative Examples 7 to 9 in which self-assembly substances were not formed, as shown in Fig. 13. By making use of reaction forming self-assembly substance with the pair of the HCPs and by detecting with PicoGreen, a signal of the DNA chip was remarkably amplified depending on the concentrations of the capture probe.

### (Examples 10 to 12 and Comparative Examples 10 to 12)

### 1. Object

The effect of signal amplification with a pair of HCPs (HCP-1 and HCP-2) was detected by using EtBr.

### 2. Materials

### (Examples 10 to 12)

a) As a capture probe, the capture DNA probe-1a prepared at 500 ng/µL (Example 10), 50 ng/µL (Example 11) or 5 ng/µL (Example 12) was used, respectively.
b) As a primary hybridization solution, the above primary hybridization solution A was used.
c) As a secondary hybridization solution, the above secondary hybridization solution C was used.

### (Comparative Examples 10 to 12)

a) As a capture probe, the capture DNA probe-1a prepared at 500 ng/µL (Comparative Example 10), 50 ng/µL (Comparative Example 11) or 5 ng/µL (Comparative Example 12) was used, respectively.
b) As a primary hybridization solution, the above primary hybridization solution A was used.
c) As a secondary hybridization solution, the above secondary hybridization solution D was used.

### 3. Methods

Each Experiment was carried out on the procedure and conditions both similar to those described in item 3 for Examples 1 to 3 and Comparative Examples 1 to 3.

### 4. Detection Method

10 µg/µL of EtBr solution was dropped onto the spot on the slide glass and left standing for 30 minutes at room temperature. The slide glass was rinsed three times with 0.5×TBE at room temperature, dried by spraying air and observed with a fluorescent microscope (580 nm).

### 5. Results

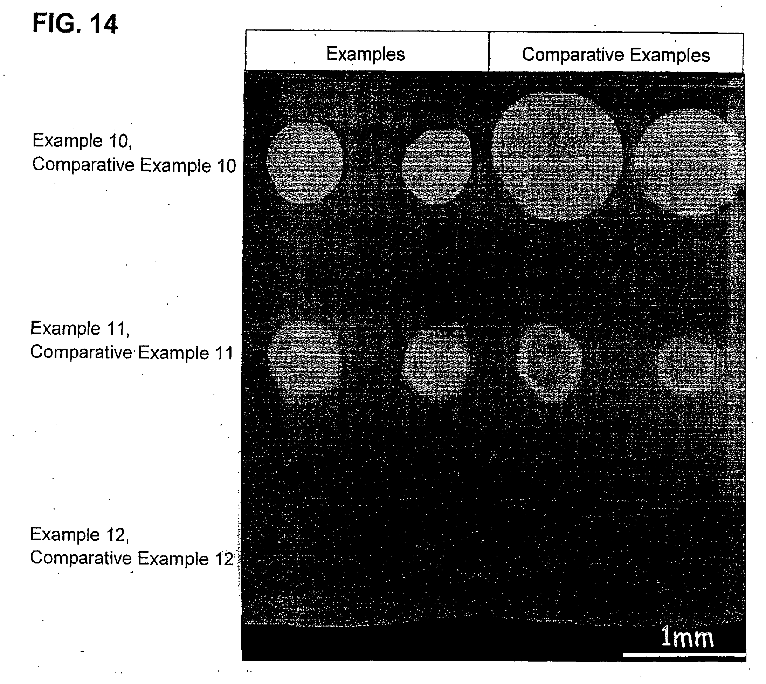

The results of Examples 10 to 12 and Comparative Examples 10 to 12 are shown in Fig. 14. The fluorescence intensities of Examples 10 to 12 in which self-assembly substances were formed strikingly increased in comparison with those of Comparative Examples 10 to 12 in which self-assembly substances were not formed, as shown in Fig. 14. By making use of reaction forming a self-assembly substance with the pair of the HCPs and by detecting with EtBr, a signal of the DNA chip was remarkably amplified depending on the concentrations of the capture probe.

### (Examples 13 to 15 and Comparative Examples 13 to 15)

### 1. Object

The effect of signal amplification was examined by using a pair of HCPs (HCP-3 and HCP-4) labeled with Cy3 and different from HCPs used in Examples 1 to 3.

### 2. Materials

### (Examples 13 to 15)

a) As a capture probe, the capture DNA probe-1a prepared at 500 ng/µL (Example 13), 50 ng/µL (Example 14), or 5 ng/µL (Example 15) was used, respectively.
b) As a primary hybridization solution, the above primary hybridization solution A was used.
c) As a secondary hybridization solution, 25 pmoles of Cy3-labeled HCP-3 and 25 pmoles of Cy3-labeled HCP-4 were dissolved in 10 µL of the hybrid solution, followed by heating for 2 minutes at 95°C to prepare a secondary hybridization solution E.

### (Comparative Examples 13 to 15)

a) As a capture probe, the capture DNA probe-1a prepared at 500 ng/µL (Comparative Example 13), 50 ng/µL (Comparative Example 14) or 5 ng/µL (Comparative Example 15) was used, respectively.
b) As a primary hybridization solution, the above primary hybridization solution A was used.
c) As a secondary hybridization solution, 25 pmoles of Cy3-labeled probe 5 was dissolved in 10 µL of the hybrid solution, followed by heating for 2 minutes at 95°C to prepare a secondary hybridization solution F.

### 3. Methods

The capture probe was applied onto a slide glass by spotting to make the probe immobilized. Then, 25 µL of the primary hybridization solution was dropped onto the spot on the slide glass, covered with a cover glass, and left standing for 2 hours at 42°C in a hybridization chamber. After that, the slide glass was taken out, and the cover glass was removed immersing in the solution of 2×SSC and 0.1% SDS at room temperature, followed by rinsing of the slide glass with the solution of 2×SSC and 0.1% SDS. In addition, the slide glass was left standing in solutions of 1×SSC and 0.2×SSC for 2 minutes, respectively, at room temperature and dried by spraying air.

Then 10 µL of the secondary hybridization solution was dropped to the spot on the slide glass, covered with a cover glass, and left standing for 6 hours at 54°C in a hybridization chamber. After that, the slide glass was taken out; and the cover glass was removed immersing in the solution of 2×SSC and 0.1% SDS at room temperature, followed by rinsing of the slide glass with the solution of 2×SSC and 0.1% SDS. Further, the slide glass was left standing in solutions of 1×SSC and 0.2×SSC for 2 minutes, respectively, at room temperature and then dried by spraying air.

### 4. Detection Method

The above slide glass was observed with a fluorescent microscope (580 nm).

### 5. Results

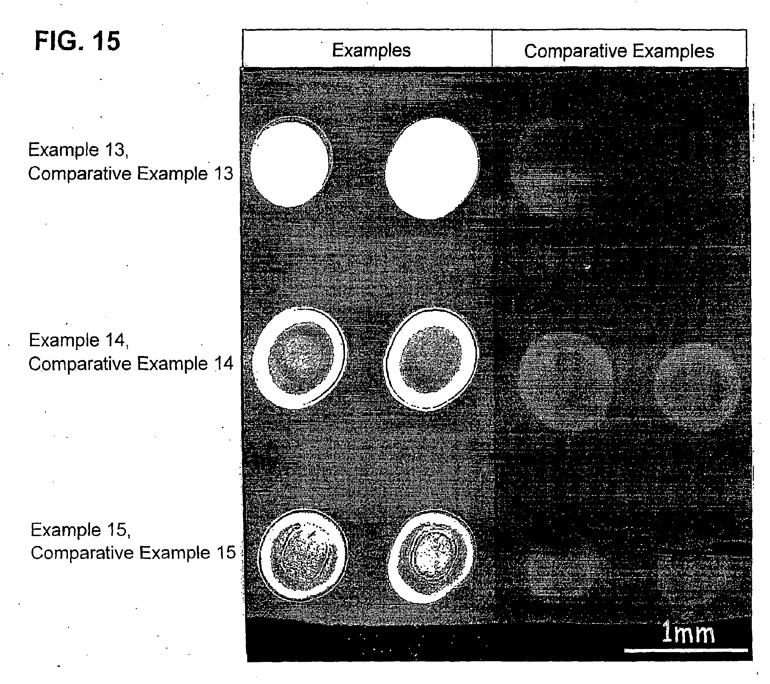

The results of Examples 13 to 15 and Comparative Examples 13 to 15 are shown in Fig. 15. The fluorescence intensities of Examples 13 to 15 in which self-assembly substances were formed strikingly increased in comparison with those of Comparative Examples 13 to 15 in which self-assembly substances were not formed, as shown in Fig. 15. By making use of reaction forming a self-assembly substance with the pair of the Cy3-labeled HCPs, a signal of the DNA chip was remarkably amplified depending on the concentrations of the capture probe. Compared to Examples 1 to 3, it was found that the sensitivity for detection could be improved by adjusting base sequences of a pair of HCPs and the reaction conditions.

### (Examples 16 to 18 and Comparative Examples 16 to 18)

### 1. Object

The effect of signal amplification was examined by using a pair of dimer-forming probes labeled with Cy3 and a pair of cross-linking probes.

### 2. Materials

### (Examples 16 to 18)

a) As a capture probe, the capture DNA probe-1 prepared at 500 ng/µL. (Example 16), 50 ng/µL (Example 17), or 5 ng/µL (Example 18) was used, respectively.
b) As a primary hybridization solution, the above primary hybridization solution A was used.
c) A secondary hybridization solution was prepared as follows: 25 pmoles of Cy3-labeled dimer-forming probe-1 and 25 pmoles of Cy3-labeled dimer-forming probe-2 were dissolved in 10 µL of the hybrid solution, heated for 2 minutes at 95°C, and then left standing for 16 hours at 45°C. To this was added 0.5 µL each of 50 µM cross-linking probe-1 and 50 µM cross-linking probe-2 solutions to prepare a secondary hybridization solution G.

### (Comparative Examples 16 to 18)

a) As a capture probe, the capture DNA probe-1a prepared at 500 ng/µL (Comparative Example 16), 50 ng/µL (Comparative Example 17), or 5 ng/µL (Comparative Example 18) was used, respectively.
b) As a primary hybridization solution, the above primary hybridization solution A was used.
c) A secondary hybridization solution was prepared as follows: 25 pmoles of Cy3-labeled dimer-forming probe-1 and 25 pmoles of Cy3-labeled dimer-forming probe-2 were dissolved in 10 µL of the hybrid solution, heated for 2 minutes at 95°C, and then left standing for 16 hours at 45°C. To this was added 0.5 µL of distilled water to prepare a secondary hybridization solution H.

### 3. Methods

The capture probe was applied onto a slide glass by spotting to make the probe immobilized. Then, 25 µL of the primary hybridization solution was dropped onto the spot on the slide glass, covered with a cover glass, and left standing for 2 hours at 42°C in a hybridization chamber. After that, the slide glass was taken out, and the cover glass was removed immersing in the solution of 2xSSC and 0.1% SDS at room temperature, followed by rinsing of the slide glass with the solution of 2×SSC and 0.1% SDS. In addition, the slide glass was left standing in solutions of 1×SSC and 0.2×SSC for 2 minutes, respectively, at room temperature and then dried by spraying air.

To the spot on the slide glass was then dropped 10 µL of the secondary hybridization solution, covered with a cover glass, and left standing for 2 hours at 58°C in a hybridization chamber. After that, the slide glass was taken out, and the cover glass was removed immersing in the solution of 2×SSC and 0.1% SDS at room temperature, followed by rinsing with the solution of 2×SSC and 0.1% SDS. Further, the slide glass was left standing in solutions of 1×SSC and 0.2×SSC for 2 minutes, respectively, at room temperature and then dried by spraying air.

### 4. Detection Method

The slide glass was observed with a fluorescent microscope (580 nm).

### 5. Results

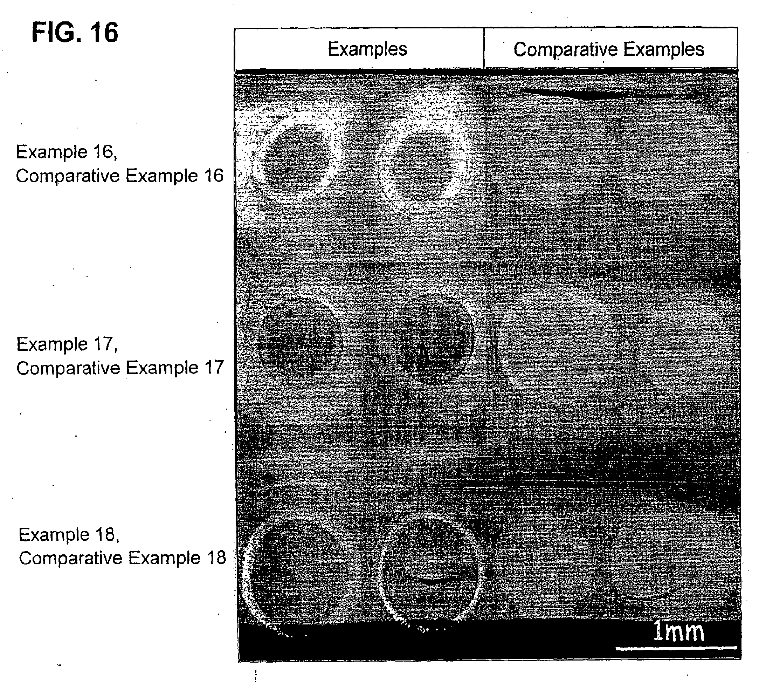

The results of Examples 16 to 18 and Comparative Examples 16 to 18 are shown in Fig. 16. The fluorescence intensities of the Examples 16 to 18 in which self-assembly substances were formed strikingly increased in comparison with those of Comparative Examples 16 to 18 in which self-assembly substances were not formed, as shown in Fig. 16. By making use of reaction forming a self-assembly substance by the use of the pair of the Cy3-labeled dimer-forming probes and the pair of the cross-linking probes, a signal of the DNA chip was remarkably amplified depending on the concentrations of the capture probe.

### (Experimental Examples 1 to 4)

### 1.Object

Specificity of signal amplification by HCP was examined by using two kinds of pairs of Cy3-labeled HCPs combinations (HCP-3 and HCP-4 pair, HCP-5 and HCP-6 pair).

### 2. Materials

a) As a capture probe, either the capture DNA probe-2a having a region complementary to the target gene-2 or the capture DNA probe-1b having a region complementary to the target gene-1 was prepared each at 50 pmoles/µL and 5 pmoles/µL.
b) As a primary hybridization solution in Experimental Example 1, the following primary hybridization solution B was prepared for use: 3 pmoles of the target gene-1 was dissolved in 30 µL of hybridization solution X (5×Denhardt's solution, 0.1 mg/mL Salmon sperm DNA, 0.2% SDS, 6×SSC), and heated for 2 minutes at 95°C to prepare the primary hybridization solution B.
   As a primary hybridization solution in Experimental Example 2, the following primary hybridization solution C was prepared for use: 3 pmoles of the target gene-2 was dissolved in 30 µL of the hybridization solution X, and heated for 2 minutes at 95°C to prepare the primary hybridization solution C.
   As a primary hybridization solution in Experimental Example 3, the following primary hybridization solution D was prepared for use: 3 pmoles each of the target gene-1 and the target gene-2 was dissolved in 30 µL of the hybridization solution X, and heated for 2 minutes at 95°C to prepare the primary hybridization solution D.
   As a primary hybridization solution in Experimental Example 4, the following primary hybridization solution E was prepared for use: 30 µL of the hybridization solution X without adding any target gene was heated for 2 minutes at 95°C to prepare the primary hybridization solution E.
c) As a secondary hybridization solution, 30 pmoles each of Cy3-labeled HCP-5, Cy3-labeled HCP-6, Cy3-labeled HCP-3 and Cy3-labeled HCP-4 were dissolved together in 30 µL of the hybridization solution X and heated for 2 minutes at 95°C to prepare the secondary hybridization solution X.

### 3. Methods

The capture DNA probe-2a (50 pmoles/µL or 5 pmoles/µL) or the capture DNA probe-1b (50 pmoles/µL or 5 pmoles/µL) was applied onto a slide glass by spotting, respectively, to make the probes immobilized. In a similar way, 25 µL each of the primary hybridization solutions B to E was dropped onto each of the four spotted slide glasses in a chamber for *in situ* PCR, and the slide glasses were shielded and left standing for 2 hours at 42°C. After that, the slide glasses were removed from the chamber and rinsed twice with the solution of 2×SSC and 0.1% SDS at room temperature. In addition, the slide glasses were left standing successively in 1×SSC and 0.2×SSC for 2 minutes each and dried by spraying air.

Next, 25 µL each of the secondary hybridization solutions was dropped onto the slide glasses in the *in situ* PCR chamber, and the slide glasses were shielded and left standing for 2 hours at 57.7°C. After that, the slide glasses were removed from the chamber and rinsed twice with the solution of 2×SSC and 0.1% SDS at room temperature. In addition, the slide glasses were left standing in 1×SSC and 0.2×SSC for 2 minutes each, dried by spraying air and observed with a fluorescent microscope (580 nm).

### 4. Results

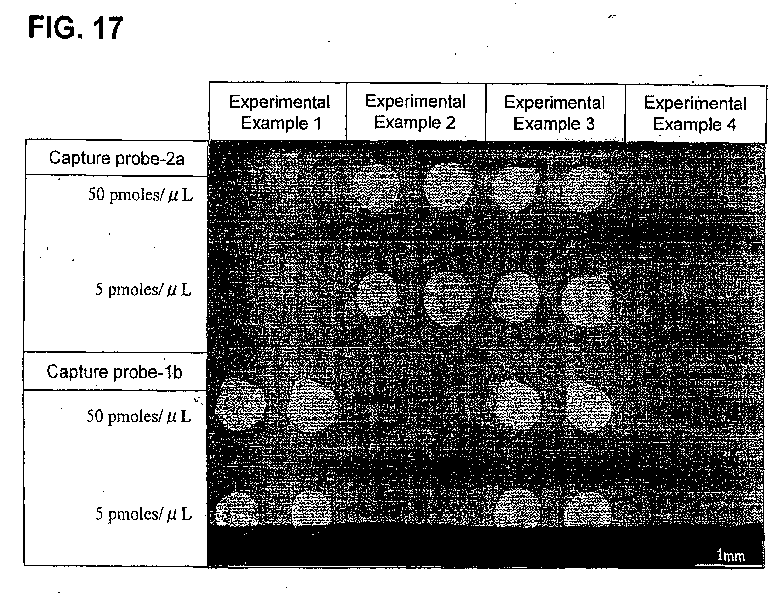

The results of Experimental Examples 1 to 4 are shown in Fig. 17. As demonstrated in Fig. 17, the target gene-1 was captured where the capture DNA probe-1b was immobilized, and its signals were amplified by HCPs, while the target gene-2 was captured where the capture DNA probe-2a was immobilized, and its signals were amplified by HCPs.

### (Experimental Examples 5 and 6)

### 1. Object

By using a gold electrode which can measure an electric current by differential pulse voltammetry as a support, signal amplification of a gene by HCPs was examined with an electrochemically active intercalator in place of a fluorescent intercalator.

### (Experimental Example 5)

### 2. Materials and Methods

A gold electrode (manufactured by BAS Inc.) was polished with 0.05 µm of a suspension of alumina powder (manufactured by Baikalox Corp.), rinsed with sterile distilled water and cleaned by sonication in 100% ethanol for 15 minutes. After rinsing again with sterile distilled water, the surface of Au was dried, and then 2 µL of a solution of the capture DNA probe-2b prepared at a concentration of 10 pmoles/µL was applied onto the Au. The electrode was capped in order to prevent from drying and left standing overnight in a humid chamber at room temperature.

The Au electrode was rinsed with sterile distilled water and immersed in a measuring buffer [0.1 M acetic acid buffer (pH 5.6, potassium acetate/acetic acid), 0.1 M potassium chloride, 50 µM Hoechist33258] for 180 seconds, and its current intensity (a value for the capture probe: io) was measured by differential pulse voltammetry using ALS630 (manufactured by BSA Co. Ltd.).

As a target solution, 5xSSC solution containing the target gene-2 at a concentration of 1 pmole/µL was prepared. After the Au electrode had been rinsed with distilled water, 2 µL of the above target solution was applied onto the Au electrode.

After having been capped, the electrode was transferred into a humid chamber and left standing for 180 minutes at 45°C for hybridization, followed by rinsing once with the solution of 2×SSC and 0.1% SDS, twice with a solution of 0.2×SSC and 0.1% SDS, once with 1×SSC, and once with 0.2×SSC and drying.

The electrode was immersed in the measuring buffer [0.1 M acetic acid buffer (pH 5.6, potassium acetate/acetic acid), 0.1 M potassium chloride, 50 µM Hoechist33258] for 180 seconds, and then its current intensity was measured by differential pulse voltammetry (a value when the target gene is bound to the capture probe: i₁).

After the Au electrode was rinsed with sterile distilled water, 2 µL of a solution containing a pair of HCPs [0.156 pmoles/µL each of HCP-5 and HCP-6, 12×SSC, 10 µM Hoechist33258] was applied onto the Au electrode.

After having been capped, the electrode was transferred into a humid chamber and left standing for 120 minutes at 58°C to form a self-assembly substance.

The electrode was rinsed once with the solution of 2×SSC and 0.1% SDS, twice with the solution of 0.2×SSC and 0.1% SDS, once with 1×SSC, and twice with 0.2×SSC, dried and immersed in the measuring buffer [0.1M acetic acid buffer (pH 5.6, potassium acetate/acetic acid), 0.1 M potassium chloride, 50 µM Hoechist33258] for 180 seconds, and then its current intensity was measured by differential pulse voltammetry (a value when the HCPs self-assembled on the target gene bound to the capture probe: i₂).

### (Experimental Example 6)

Experiments were carried out according to the procedure and conditions similar to those described above for Experimental Example 5 except that as a target solution, a 5×SSC solution containing the target gene-2 at a concentration of 0.5 pmoles/µL was prepared for use, in place of the 5×SSC solution containing the target gene-2 at a concentration of 1 pmole/µL.

### 5. Results

Table 1 shows the results of Experimental Example 5 (target gene-2; 1 pmole/µL, and Table 2 shows the results of Experimental Example 6 (target gene-2; 0.5 pmoles/µL).

In both Table 1 and Table 2, the rate of increase in peak value of current observed was larger when self-assembly was effected with the use of HCPs, compared to when only the target gene was hybridized, as shown in the results of Tables 1 and 2.

**Table 1**

| Target gene-2; 1 pmole/µL | | | | |
|---|---|---|---|---|
| 1 pmole/µL | Current value (A) | Increment (Δi) | Rate of Increase (%) | |
| Peak value i₀ | -5.60E-07 | | | |
| ↓ | | -1.91E-07 | 34.1 | Δi₀ → i₁ |
| Peak value i₁ | -7.51E-07 | | | |
| ↓ | | -9.02E-07 | 120.1 | Δi₁ → i₂ |
| Peak value i₂ | -1.65E-06 | | | |
| Total | | -1.09E-06 | 195.2 | Δi₀ → i₂ |

**Table 2**

| Target gene-2; 0.5 pmoles/µL | | | | |
|---|---|---|---|---|
| 1 pmole/µL | Current value (A) | Increment (Δi) | Rate of Increase (%) | |
| Peak value i₀ | -5.72E-07 | | | |
| ↓ | | -1.16E-07 | 20.3 | Δi₀ → i₁ |
| Peak value i₁ | -6.88E-07 | | | |
| ↓ | | -1.13E-06 | 164.2 | Δi₁→ i₂ |
| Peak value i₂ | -1.82E-06 | | | |
| Total | | -1.25E-06 | 217.8 | Δi₀→ i₂ |

### Capability of Exploitation in Industry:

As described above, the signal amplification method for a DNA chip of the present invention using a self-assembly reaction of oligonucleotides can improve simply and remarkably sensitivity for detection of a target gene on the DNA chip without any complicated procedures.

## Claims

1. A signal amplification method for a DNA chip, wherein sensitivity for detection of a target gene on the DNA chip is improved by making use of a self-assembly reaction which forms a double-stranded self assembly substance having a regular higher-order structure of oligonucleotides.

2. The signal amplification method according to claim 1, wherein the self-assembly reaction comprises the steps of:
providing a plurality of pairs of oligonucleotide-probes comprising n (n≧3) regions, each region of a first probe of the pair of probes being complementary to each region of a second probe of the pair of probes; and
hybridizing the pairs of oligonucleotide-probes such that the first probes and the second probes cross each other in alternation,
wherein the oligonucleotide-probes are self-assembled to form the double-stranded self-assembly substance.

3. The signal amplification method according to claim 1, wherein the self-assembly reaction comprises the steps of
providing a first group and a second group,
the first group including a plurality of pairs of dimer-forming probes containing a pair of an oligonucleotide No.1 and an oligonucleotide No.2, each oligonucleotide having three regions of a 3' side region, a mid-region and a 5' side region, in which the mid-regions thereof have base sequences complementary to each other to form a dimer probe, and the 3' side regions and the 5' side regions thereof have base sequences not complementary to each other,
the second group including a plurality of pairs of cross-linking probes containing a pair of an oligonucleotide No.3 and an oligonucleotide No.4, each oligonucleotide having two regions of a 3' side region and a 5' side region, in which the 3' side regions and the '5' side regions thereof have, base sequences not complementary to each other, and the pairs of the cross-linking probes having base sequences capable of cross-linking the dimer probes formed from the dimer-forming probes; and
hybridizing the probes,
wherein the oligonucleotides are self-assembled to form the self assembly substance.

4. The signal amplification method according to claim 3, wherein the base sequences of the probes are made complementary to each other in the following respective pairs:
the 3' side region of the oligonucleotide No.1 in the first group and
the 3' side region of the oligonucleotide No.3 in the second group;
the 5' side region of the oligonucleotide No. 2 in the first group and
the 5' side region of the oligonucleotide No.4 in the second group;
the 3' side region of the oligonucleotide No.4 in the second group and
the 3' side region of the oligonucleotide No.2 in the first group; and
the 5' side region of the oligonucleotide No.3 in the second group and
the 5' side region of the oligonucleotide No.1 in the first group.

5. The signal amplification method according to claim 3, wherein the base sequences of the probes are made complementary to each other in the following respective pairs:
the 3' side region of the oligonucleotide No.1 in the first group and
the 3' side region of the oligonucleotide No.3 in the second group;
the 5' side region of the oligonucleotide No.2 in the first group and
the 5' side region of the oligonucleotide No.3 in the second group;
the 3' side region of the oligonucleotide No.2 in the first group and
the 3' side region of the oligonucleotide No.4 in the second group; and
the 5' side region of the oligonucleotide No.1 in the first group and
the 5' side region of the oligonucleotide No.4 in the second group.

6. The signal amplification method according to any one of claims 1 to 5, wherein the DNA chip comprises a support to bind a gene for capturing the target gene, and the support is of micro plate type, slide glass type, microparticle type or electroconductive substrate type.

7. The signal amplification method according to any one of claims 1 to 6, wherein a single-stranded DNA and/or RNA are used as the target gene.

8. The signal amplification method according to any one of claims 1 to 6, wherein a double-stranded DNA and/or RNA are used as the target gene.

9. The signal amplification method according to any one of claims 1 to 6, wherein SNPs (single nucleotide polymorphisms) are used as the target gene.

10. The signal amplification method according to any one of claims 1 to 9, wherein a base sequence of the oligonucleotides used for the self-assembly reaction is made, in advance, complementary to a base sequence of the target gene.

11. The signal amplification method according to any one of claims 1 to 9, wherein in order to link the target gene to the self-assembly substance, there is used a joint-probe which contains a first region complementary to a base sequence of the target gene and a second region complementary to a base sequence of the oligonucleotides used for formation of the self-assembly substance.

12. The signal amplification method according to any one of claims 1 to 11, wherein by hybridizing a labeled probe to the self-assembly substance formed by the self-assembly reaction of the oligonucleotides and bound to the target gene, the presence of the self-assembly substance is detected.

13. The signal amplification method according to claim 12, wherein the labeled probe is a probe labeled with an enzyme of color generation type, an enzyme of luminescence generation type or a radioisotope.

14. The signal amplification method according to any one of claims 1 to 11, wherein the presence of the self-assembly substance is detected by:
adding a fluorescent substance capable of binding to a nucleic acid to the self-assembly substance; and
measuring a photochemical change of the fluorescent substance.

15. The signal amplification method according to any one of claims 1 to 11, wherein the presence of the self-assembly substance is detected by:
labeling in advance at least one of the oligonucleotides forming the self-assembly substance with a fluorescence substance; and
measuring a photochemical change of the fluorescent substance.

16. The signal amplification method according to any one of claims 1 to 11, wherein the presence of the self-assembly substance is detected by:
labeling in advance at least one of the oligonucleotides forming the self-assembly substance with a radioisotope; and
detecting the radioisotope.

17. The signal amplification method according to any one of claims 1 to 11, wherein the presence of the self-assembly substance is detected by:
labeling in advance at least one of the oligonucleotides forming the self-assembly substance with an enzyme of color generation type or an enzyme of luminescence generation type; and
measuring a photochemical change due to the enzyme.

18. The signal amplification method according to any one of claims 1 to 17, wherein the oligonucleotides are comprised of at least one base selected from the group consisting of DNA, RNA, PNA and LNA.


SEQUENCE LISTING

<110> Sanko Junyaku Co., Ltd.

<120> Signal Amplification Method for DNA chip

<130> 76161-PCT-EP

<140> PCT/JP02/10034
<141> 2002-09-27

<150> JP 2001-303816
<151> 2001-09-28

<160> 16

<210> 1
<211> 30
<212> DNA
<213> Artificial Sequence

<220>
<221> misc_feature
<222> (1)
<223> Amino group attached at the 5'end

<220>
<223> SEQ ID#:1

<400> 1
aaaaaagcgg gaaatcgtgc gtgacattaa 30

<210> 2
<211> 80
<212> DNA
<213> Artificial Sequence

<220>
<223> SEQ ID#:2

<400> 2
cgtggccatc tcttgctcga agtccagggc gacgtagcac agcttctcct taatgtcacg 60

cacgatttcc cgctcggccg 80

<210> 3
<211> 75
<212> DNA
<213> Artificial Sequence

<220>
<223> SEQ ID#:3

<400> 3
cctggacttc gagcaagaga tggccggcac caccatgtac cctggcattg gtccaccgca 60

aatgcttcta ggcgg 75

<210> 4
<211> 75
<212> DNA
<213> Artificial Sequence

<220>
<223> SEQ ID#:4

<400> 4
ggccatctct tgctcgaagt ccaggcaatg ccagggtaca tggtggtgcc ccgcctagaa 60

gcatttgcgg tggac 75

<210> 5
<211> 60
<212> DNA
<213> Artificial Sequence

<220>
<223> SEQ ID#:5

<400> 5
ccctggactt cgagcaagag cgtggacatc cgcaaagacc gcaggagtat gacgagtccg 60

<210> 6
<211> 60
<212> DNA
<213> Artificial Sequence

<220>
<223> SEQ ID#:6

<400> 6
ctcttgctcg aagtccaggg ggtctttgcg gatgtccacg cggactcgtc atactcctgc 60

<210> 7
<211> 60
<212> DNA
<213> Artificial Sequence

<220>
<223> SEQ ID#:7

<400> 7
gacttcgagc aagagatggc cctgataggg tgcttgcgag ccattggcaa tgagcggttc 60

<210> 8
<211> 60
<212> DNA
<213> Artificial Sequence

<220>
<223> SEQ ID#:8

<400> 8
gagcatcccc caaagttcac ctcgcaagca ccctatcagg gtggcatcca cgaaactacc 60

<210> 9
<211> 40
<212> DNA
<213> Artificial Sequence

<220>
<223> SEQ ID#:9

<400> 9
gccatctctt gctcgaagtc gaaccgctca ttgccaatgg 40

<210> 10
<211> 40
<212> DNA
<213> Artificial Sequence

<220>
<223> SEQ ID#:10

<400> 10
gtgaactttg ggggatgctc ggtagtttcg tggatgccac                    40

<210> 11
<211> 50
<212> DNA
<213> Artificial Sequence

<220>
<221> misc_feature
<222> (1)
<223> Amino group attached at the 5'end

<220>
<223> SEQ ID#:11

<400> 11
gatcagacac ttcaaggtct ctggcgcttt aagattagcc tgcgtccgat          50

<210> 12
<211> 40
<212> DNA
<213> Artificial Sequence

<220>
<221> misc_feature
<222> (1)
<223> Amino group attached at the 5'end

<220>
<223> SEQ ID#:12

<400> 12
aaaaaagcgg gaaatcgtgc gtgacattaa ggagaagctg                    40

<210> 13
<211> 100
<212> DNA
<213> Artificial Sequence

<220>
<223> SEQ ID#:13

<400> 13
tgcttcttct gtgggtaacg tccagttaat cagctcttaa cctatcaacc ctccaactag   60

ctaatcggac gcaggctaat cttaaagcgc caggcccgaa                    100

<210> 14
<211> 69
<212> DNA
<213> Artificial Sequence

<220>
<223> SEQ ID#:14

<400> 14
gagggttgat aggttaagag ctggtagaga tcggaaggaa caccagcagc aaacgcgata 60

agttgaccg 69

<210> 15
<211> 69
<212> DNA
<213> Artificial Sequence

<220>
<223> SEQ ID#:15

<400> 15
cagctcttaa cctatcaacc ctcctggtgt tccttccgat ctctaccggt caacttatcg 60

cgtttgctg 69

<210> 16
<211> 50
<212> DNA
<213> Artificial Sequence

<220>
<221> misc_feature
<222> (1)
<223> Mercapto group attached at the 5'end

<220>
<223> SEQ ID#:16

<400> 16
gatcagacac ttcaaggtct ctggcgcttt aagattagcc tgcgtccgat 50